(19) Europäisches Patentamt
European Patent Office
Office européen des brevets

(11) **EP 2 471 380 A2**

(12) **EUROPEAN PATENT APPLICATION**

(43) Date of publication:
04.07.2012 Bulletin 2012/27

(21) Application number: 12162209.6

(22) Date of filing: 13.11.2007

(51) Int Cl.:
*A23K 1/18* (2006.01)     *A23K 1/14* (2006.01)
*A23K 1/16* (2006.01)     *A23K 1/10* (2006.01)
*A61K 36/48* (2006.01)    *A61K 36/064* (2006.01)
*A61K 36/28* (2006.01)    *A61K 38/02* (2006.01)

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(30) Priority: 13.11.2006 GB 0622582

(62) Document number(s) of the earlier application(s) in
accordance with Art. 76 EPC:
09010213.8 / 2 119 371
07824547.9 / 2 091 352

(71) Applicant: Biorigin Scandinavia AS
0253 Oslo (NO)

(72) Inventor: Elvebø, Odd
1475 Finstadjordet (NO)

(74) Representative: Gardner, Rebecca Katherine
Dehns
St Bride's House
10 Salisbury Square
London EC4Y 8JD (GB)

Remarks:
This application was filed on 29-03-2012 as a
divisional application to the application mentioned
under INID code 62.

(54) **Animal feeds and veterinary compositions**

(57) The present invention relates to glucan or mannan for use in ameliorating the side-effects of *Fabaceae* protein in an animal diet, whereby the glucan or mannan is administered to an animal together with or separately from said *Fabaceae* protein.

Figure 2:

B  Total dry matter intake

## Description

The present invention relates to an animal feed and/or feed ingredient or feed additive for weight stabilization or gain in animals.

The invention also relates to feed or other formulations of use in therapy, in particular in treating or preventing bowel disease or diarrhoea and improving bowel health.

In addition the present invention defines new methods for killing ectoparasites and/or preventing or inhibiting the adhesion of ectoparasites to an animal as well as the treatment and prevention of ectoparasite-related diseases and conditions and the prevention or reduction of infestation and infection of animals by ectoparasites.

The animal feed and farming industries are continuously looking for new animal feed compositions which are efficient and may be used to treat different conditions and/or to promote weight increase. A critical driver in both industries is the cost of these feed compositions. Therefore there is increasing interest and need to replace high-cost raw materials presently used in animal feeds with low-cost materials providing the same results with regard to weight gain and treatment of different conditions.

Animal proteins are good protein sources with low prices, which can be used to partially replace fish meal. However, due to the occurrence of conditions like BSE, consumers, producers and scientists are questioning these inter-species feeding practices. Another prominent reason for replacing feeds like fish meal is the fact that aquaculture is continuously increasing and the supplies from fisheries stabilize or even tend to decrease. Fish meal is not only consumed by the fish farming industry but also in large amounts by the land farming industry and demand from this customer group is constantly rising. Furthermore, the contamination of certain fish meal with dioxins has reduced the quality of fish meal as a raw material to be included in fish feed. Those factors are forcing feed industries to search for alternative protein sources in animal feeds.

A presently dominating issue is the use of plant proteins like soy as a nutritional ingredient in feeds for fish and other animals, e.g. mammals. Soy products are valuable ingredients in feeds for carnivorous, omnivorous and herbivorous fish and other animals, because of their low price, high content of available protein with a well balanced amino acid profile, constant composition and steady supply.

In recent years, the concentration of' plant proteins in animal feeds has increased dramatically leading to unwanted side effects like inflammations and other conditions and, as a result, a reduction of weight gain, feed conversion and efficiency, and the performance of farmed animals. Plant proteins contain substances, like a broad spectrum of antinutritive factors (ANF), which stress the fish physiology and lead to adverse reactions in the animal's digestive system. That seems to reduce the animal's ability for nutrient utilization. These substances further lead to enhanced mortality and therefore economic loss for the industry. The most critical of these antinutritive factors can be inactivated by standard heat treatment (toasting) which is a common procedure during the production of plant protein meal, here especially soy protein meal.

Plant protein meals may also induce morphologic changes in the intestines of animals, as seen when feeding soy to fish. With regard to fish, this pathogenesis is classified as a non-infectious sub-acute inflammation, characterized by increased proliferation, turnover and, as such, an increased number of immature cells in the mucosa of the digestive system. This results in a reduced reabsorption of endogenous compounds, e.g. digestive enzymes, in the mucosa and the intestinal surface area becomes reduced. The bacteria composition in the intestines is also changing. This condition may weaken the fish's resistance to disease and seems to involve immunological mechanisms which are like those similar to hypersensitive reactions. These changes represent significant challenges for the feed industry to solve when soy products are included as a major source of dietary protein. For most of these plant proteins, for instance soy meal, there seems to be a limitation in inclusion levels of 20-30% for most fish, although the usual levels are around 10% as a practical commercial limitation.

It is desirable to increase the protein concentration in the animal feed as this allows a faster weight enhancement of the animal. This is an especially important factor in the fish farming industry where protein is the main feed source.

Soy protein concentrate, as an example, has high nutritional value in feeds, and the results obtained with different purifications of soy meal and protein isolates indicate a large potential for development of specialized products suitable for being the major source of dietary protein for farmed animals including, but not limited to, mammals and fish.

Thus, there is a desire to include a meaningful amount of soy in animal, particularly fish, feeds. However, as compared to traditional diets which may be composed almost entirely of fish meal, there are performance problems with soy containing diets.

The present invention solves these problems by using a glucan and/or a mannan, in combination with plant proteins, especially soy or/and sunflower protein. Preferably the glucan and/or mannan is part of a cell wall fraction or derived from cell walls.

The preferred combination of sunflower protein and glucan/mannan provides a beneficial effect when fed to animals, particularly fish, and reduces or diminishes the negative side effects of the plant protein material. Preferably the combination provides a synergistic effect.

Another aspect of the present invention is related to protecting fish or other animals against pathogens or ectoparasites which can enter and/or attach to the body. Fish lice, as exemplified by the salmon lice, *Lepeophtheirus salmonis,* and *Caligus elongatus* is a serious problem for both wild and cultivated salmon. These parasites attach themselves to salmon and sea trout and feed off them, causing them serious distress; they multiply rapidly and are capable of spreading to other fish over large areas. Sea lice are common on adult salmon, but juvenile salmon are most badly affected. The ectoparasites inflict severe damage on the surface of the fish like big lesions, puncture wounds and open sores. In the end the fish dies or the general condition and quality of the fish does not qualify for sale. The outcome is substantial economic losses for the fish farming industry. Therefore fish lice are considered to be one of the most important problems in the farming of salmonids, especially with regard to Atlantic salmon (*salmo Salar*) and rainbow trout (*Oncorhynchus mykiss*) and other fish like, but not limited to, different species from the phylum *Chordata,* exemplified by the class *Actinoperygii* exemplified by different cod species (*Gadus sp.*)*.*

The aquaculture industry has' been using antihelminthics for treatment of ectoparasites. Bathing in formalin is a widespread treatment against many parasites, especially in fresh water, while bathing in organophosphates (metrifonate, dichlorvos, azamethiphos), pyrethroids (pyrethrum, cypermethrin, deltamethrin) or hydrogen peroxide are the most common bath treatments against e.g. sea lice (*Lepeophtheirus salmonis, Caligus elongatus*). Substances such as chitin synthesis inhibitors, diflubenzuron and teflubenzuron, ivermectin and emamectin are examples of orally-(feed)-administered substances. These agents given as injections, bath treatments or as infeed preparations can inflict severe damages to the environment and the ecosystem. These and other attempts to control this problem have had limited success, are costs-intensive and there also exist many practical operational constraints like toxicity for the user. Current approaches to minimising chemotherapeutant and antihelminthic use involve monitoring infestation levels and targeting treatment to when it is likely to be most effective. As a result, demand for an easy-to administrate, cost-effective, and environmentally friendly treatment is high.

With the present invention it is possible to reduce substantially the number of ectoparasites by adding a glucan and/or a mannan to a fish diet with a substantial amount of plant proteins. Preferably the glucan and/or mannan is part of a cell wall fraction or derived from cell walls.

It has also been shown that it is possible to reduce substantially the number of ectoparasites by giving the fish or other target animal protein from a member of the *Asteraceae* family, without the need for a glucan and/or mannan component.

There is a need for improved means to avoid the negative side effects of plant protein feeding and to control parasitic infestations in fish. The present invention addresses this need by proposing the treatment of fish by feeding plant proteins, preferably soy and sunflower proteins, to the fish and optionally the coadministration of a glucan and/or a mannan. Typically the glucan/mannan is orally administered.

Thus, in one aspect, the invention provides the use of glucan and/or mannan to ameliorate the side effects of plant protein in an animal feed or diet, whereby the glucan and/or mannan is administered to an animal together with or separately from said plant protein.

Preferably the plant protein whose side effects are ameliorated is from the family *Fabaceae,* e.g. a pea, pulse or bean, in particular soy.

As part of the above ameliroation said animal preferably exhibits improved weight gain or weight stabilization. Thus the invention also provides the use of glucan and/or mannan to ameliorate the side effects of plant protein in an animal feed or diet and thereby increase or stabilize weight in an animal, whereby the glucan and/or mannan is administered to said animal together with or separately from said plant protein.

Alternatively viewed the invention provides a method of ameliorating the side effects of plant protein in an animal feed or diet, which method comprises administering an effective amount of glucan and/or mannan to an animal together with or separately from said plant protein.

In a further aspect the present invention provides the use of a plant protein and glucan and/or mannan as a combined preparation for increasing or stabilizing weight in an animal.

The invention also provides a method of increasing or stabilizing weight in an animal which method comprises administration to said animal of an effective combination of one or more plant proteins and glucan and/or mannan.

The invention also provides an animal feed composition comprising protein from the family *Fabaceae* and protein from the family *Asteraceae.* The amount of each of these two protein types is typically nutritionally significant, and is physiologically significant in that it is sufficient to reduce the number of ectoparasites or treat diarrhoea or bowel disease. The amount of *Asteraceae* protein is sufficient to ameliorate the side effects of a high *Fabaceae* protein diet and to increase or stabilize weight in an animal as compared to an *Asteraceae* free diet. Typically each protein type is present at an amount from 10-30% e.g. 12-20%. Such figures will typically refer to the amount of meal present.

The *Fabaceae* and *Asteraceae* protein are each typically present in the form of a 'meal', a well known term in the art used to refer to the residue left after some or most of the oil from a plant, seed or bean etc. has been removed, e.g. in a crushing and solvent-extraction method.

The *Asteraceae* is preferably from the genus *Helianthus,* most preferably it is *Helianthus annus* (sunflower).

The invention further provides the use of glucan and/or mannan in the manufacture of a medicament for use in increasing or stabilizing weight in an animal, wherein said medicament is administered to said animal as part of a dietary regimen which comprises one or more plant proteins.

The invention also provides a method of treating an ectoparasitic infection in an animal which method comprises administration to said animal of an effective combination of one or more plant proteins and a glucan and/or a mannan.

The invention further provides the use of glucan and/or mannan in the manufacture of a medicament for treating an ectoparasitic infection in an animal, wherein said medicament is administered to said animal as part of a dietary regimen which comprises one or more plant proteins.

In a further aspect is provided a product comprising (a) glucan and/or mannan and (b) a plant protein, for combined, separate or sequential administration to an animal for stabilising or enhancing weight of said animal and/or treating an ectoparasitic infection in said animal.

The present invention also relates to an animal feed, feed ingredient and/or adjuvant comprising a plant protein and glucan and/or mannan for weight stabilization or enhancement in animals.

Another aspect of the present invention relates to veterinary compositions comprising a plant protein and glucan and/or mannan as well as articles and kits comprising these compositions as well as their use.

The treatment of ectoparasitic infection or infestation may include killing the ectoparasites and/or preventing or inhibiting the adhesion of ectoparasites to an animal as well as the treatment and prevention of ectoparasite related diseases and conditions in an infected animal. As used herein, unless otherwise clear from the context, 'treatment' includes prophylactic treatment, e.g. reducing or preventing initial infection or infestation by ectoparasites.

As mentioned above, proteins from *Fabaceae,* particularly soy, while economically desirable components of feed compositions cause problems. In a further aspect the present inventors have demonstrated the beneficial effect on weight enhancement and on ectoparasitic infestation of adding sunflower meal to an animal feed containing other plant proteins such as soy.

Thus the present invention also provides the use of protein from a member of the family *Asteraceae* to ameliorate the side effects of other plant proteins in an animal feed, wherein the *Asteraceae* protein is administered to said animal together with or separately from said further plant protein. Preferably the *Asteraceae* protein is sunflower protein. Preferably the *Asteraceae* is used to ameliorate side effects of plant proteins derived from legumes, e.g. from *Fabaceae,* preferably soy.

Thus, in the various aspects of the invention recited above and below, the "plant protein" will preferably be from the *Asteraceae* family unless it is clear from the context that it is the negative effects of said "plant protein" which are being addressed, in which case the plant will typically be a *Fabaceae* such as soy. There is a desire to include *Fabaceae* proteins in the feed formulations and so formulations will typically contain protein from both *Fabaceae* and *Asteraceae.*

Various documents including, for example, publications and patents, are recited throughout this disclosure. All such documents are, in relevant part, hereby incorporated by reference. The citation of any given document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

Referenced herein are trade names for components including various ingredients utilized in the present invention. The inventors herein do not intend to be limited by materials under a certain trade name. Equivalent materials (e.g., those obtained from a different source under a different name or reference number) to those referenced by trade name may be substituted and utilized in the descriptions herein.

The compositions herein may comprise, consist essentially of, or consist of any of the elements as described herein.

Preferably both a mannan and a glucan are used according to the invention.

In a preferred embodiment of the present invention there is synergy between the mannan and/or glucan and one or more of the plant proteins for weight stabilization and/or weight enhancement in animals, preferably fish. Likewise, preferably there is synergy between the mannan and/or glucan and one or more of the plant proteins in the prevention, inhibition and/or treatment of ectoparasitic infestations in animals, particularly fish.

Thus preferably in the methods and uses of the invention the glucan and/or mannan and the plant protein are present in the formulation (or in the dietary regimen) in synergistic proportions.

The term "synergy" with regard to the present invention, refers to the interaction of two or more agents in a composition according to the present invention so that their combined effect is greater than the sum of their individual effects.

As previously mentioned, the plant protein which exhibits synergy with the glucan/mannan is preferably from the family *Asteraceae*, most preferably sunflower.

The compositions of the invention do not consist of just natural whole products, e.g. a plant which may have within it protein and glucan/mannan, they comprise processed components. The glucan or mannan are typically not plant derived nor from the same species as the plant protein components.

*Asteraceae* meal will typically comprise 2-50%, preferably 5-40%, more preferably 8-30% of the total feed formulation.

Preferably the glucan and/or mannan are present in the formulations or used as part of cell wall fractions. Thus in a further embodiment the invention provides the use of a cell wall preparation, preferably from *Saccharomyces cerevisiae* to ameliorate the side effects of plant protein (e.g. soy) in an animal feed or diet, whereby the cell wall preparation is administered to an animal with or separately from said plant protein.

The mannan and/or glucan used in accordance with the present invention can be form a variety of different sources. Important sources for these components are yeasts as exemplified by *Saccharomyces cerevisiae.* The yeast cell wall consists mainly of polysaccharides made up of three sugars, glucose, mannose, and N-acetylglucosamine. The mannose polysaccharides are linked to proteins to form a mannoprotein layer mainly localized at the external surface. Since the predominant carbohydrate in these proteins is mannose, they are called manno-proteins. Mannan-oligosaccharides prevents the lectin from binding to its gut cell receptor and hence contributes to reducing lectin toxicity.

The common feature of glucans that are active immune-stimulants, and preferred for use according to the present invention, is the β-1,3-chain of glucose molecules. Such glucans are most often referred to as beta-glucans. However, beta-glucans are active immune-stimulants only if there are "branches" on the β-1,3-chain of glucose molecules. These "branches" are attached by β-1,6-linkages and may consist of single glucose molecules, as in beta-glucans from mushrooms, or chains of glucose molecules, as in the beta-glucan present in the cell wall of bakers yeast. Such branched beta-glucans are called β-1,3/1,6-glucans. Among products which are correctly defined as β-1,3/1,6-glucans, there are great variations in frequency and length of the β-1,6-linked branches.

Depending upon yeast strain and type, the glucan constitutes up to 25 % of the yeast cell wall dry weight. During the process of isolating beta-glucan from yeast the outer layer of manno-protein is removed as well as most of the inner content of the cell, leaving a "ghost" particle, or Whole Glucan Particle, constituting the'beta-glucan layer.

Brewers yeast differs in composition from bakers yeast because it is grown under anoxic conditions, resulting in a low level of beta-glucan in the cell walls. Other yeasts which provide a source for the mannan and/or glucan include *Candida* sp., *Hansenula* sp., *Histoplasma* sp., *Kloeckera* sp., *Kluyveromyces* sp., *Pichia* sp., *Rhodotorula* sp., *Saccharomyces* sp., *Schizophyllum* sp., *Schizosaccharomyces* sp., *Torula* sp. and *Torulopsis* sp..

Another source of mannan and/or glucan are mushrooms or fungi exemplified by those belonging to the classes *Mastigomycotina, Ascomycotina, Basidiomycotina,* and *Deuteromycotina* (imperfect fungi). Other suitable fungi include *Aspergillus* sp., *Penicillium* sp., *Sclerotinia* sp., and *Sclerotum* sp. They have the beta-1,3/1,6-glucans scleroglucan, lentinan and schizophyllan which are extracted from medicinal mushrooms, and are active immune-stimulants.

A third source of mannan and/or glucan are the members of the *Gramineae* (grasses), amongst the Angiosperms, where they are major components of endosperm walls of commercially important cereals such as oat, barley, rye, sorghum, rice and wheat. Apart from these, plants are not preferred sources.

Preferred glucans have a 1,3-linked backbone.

A fourth source of mannan and/or glucan are algae, exemplified by the classes *Chlorophyceae, Charophyceae Euglenophyceae, Phaeophyceae, Bacillariophyceae, Chrysophyceae, Xanthophyceae, Pyrrophyceae* and *Rhodophyceae.* Laminarin is an example for a glucan product from sea-weed.

It is also possible to derive said mannan and/or glucan from the cell walls of Bacteria like *Alcaligenes* (*Achromobacteriaceae*)*, Agrobacterium* and *Rhizobium* (*Rhizobacteriaceae*). Examples are *Alcaligenes faecalis, Agrobacterium radiobacter* and A. *rhizogenes, Rhizobium japonicum og R. trifolii, Streptococcus pneumoniae* as well as the *Cyanobacteriaceae Anabaena cylindrica.*

The mannan and/or glucan, i.e.'the carbohydrate components, can be used in different forms, untreated or treated/processed. In the animal feed and farming, industry the cells of organisms, most often yeast cells, are used, and fed directly to the animals. These products come in different forms and shapes, like compressed, liquid, crumbled, dry, active, in-active cells , and combinations like active dry, instant active dry and inactive dry. These products are most often the remnants of the cells used for other production processes like brewing.or baking.

Thus the glucan/mannan component can be particulate or soluble or in any physical state between a particulate and soluble product.

In addition to direct feeding of sources of mannan and/or glucan, the industries use processed products or cell extracts. These products may be hydrolysed or autolysed cells, partially or completely purified cell walls, isolated cell components other then the cell walls, isolated sources or a mixture of isolated cell or sources, either used as focused end result of the production process or by mixing these components to ready-to-use formulas. Another usual application is to mix the'yeast cell or one or more yeast cell components with other ingredients after their extraction or simply use them together. All these products are available in various forms suited to different types of use: liquid, semi-paste, paste, fine powder, oil-coated powder, microgranulated powder, to mention only some.

Products containing isolated carbohydrate components may be combination products of two or more components (e.g. from the yeast cell wall), for example a combination of glucans and mannans.

The carbohydrate component may be mixed with other agents not being part of the cell walls, like vitamins or minerals. Examples of this group of products are mixtures of beta-glucans, mannose and peptidoglycans; glucan-products combined with minerals and vitamins as well as mixtures of beta-glucans, nucleotides, mannose, vitamins, minerals and other components.

Preferred glucans are those derived from yeast cell walls which have been treated by acid or enzyme to significantly reduce or eliminate (1,6) linkages within the glucan branches (a single (1,6) link is required to form the branch). Thus, preferably less than 10%, more preferably less than 5%, most preferably less than 3% or 2% of the glycosidic bonds in the molecule will be (1,6) linkages.

Preferably the glucan component of a soluble glucan has a numerical average molecular weight of each single glucan chain from about 10 kDa to 30 kDa, preferably on average 20 kDa (+/- 5 kDa) and multiple chain MW in aqueous solution from about 500 kDa to about 1500 kDa.

Preferred glucans of the invention have a beta-1,3 backbone, i.e. the backbone is made up of beta-1,3 linked glucopyranose units. These preferred glucans have one or more beta-1,3 side chains, i.e. side chains attached to the backbone via a beta-1,6 linkage and where the side chains are made up of beta-1,3 linked glucopyranose units. The side chain comprises 2 or more, typically 3, 4, 5, 10 or more beta-1,3 linked glucopyranose units.

Mannan is a polysaccharide containing a high proportion of mannose sub-units. Preferably it is made up of D-mannose, D-glucose and D-galactose at a ratio of approximately 3:1:1.

The most preferred source for the glucan and/or mannan for the weight stabilization/enhancement application are cell walls from *Saccharomyces cerevisiae.* Of these, a preferred source for use in the present invention is the yeast product PatoGard® as sold by Immunocorp, a Norwegian based company. The composition of said product is as follows:

| Component | weight % |
|---|---|
| Carbohydrate: | min 40 |
| Protein: | max 32 |
| Ash: | max 8 |
| Lipids: | max 15 |
| Moisture: | max 8 |

Typical values for the carbohydrate components are as follows:

| Component | % of total carbohydrates |
|---|---|
| Glucan | 20 |
| Mannan | 25 |
| Chitin | < 1 |
| Glycogen | < 2 |

The most preferred source for the glucan and/or mannan component for the ectoparasitic applications are cell walls from *Saccharomyces cerevisiae.* Of these, a preferred source is the hydrolyzed yeast product MacroGard® Feed Ingredient as sold by Immunocorp, a Norwegian based company. The composition of said product is as follows:

| COMPOSITION | % by weight | typical range |
|---|---|---|
| | | |
| CARBOHYDRATES | min 60 | 63-68 |
| LIPIDS | max 18 | 13-18 |
| PROTEIN | max 8 | 5-7 |
| ASH | max 12 | 6-10 |
| TOTAL SOLID | min 92 | 94-97 |

An alternative is MacroGard®Pet which has the following composition:

| Component | % by weight | % Typical range by weight |
|---|---|---|
| Carbohydrates | min 65 | 65-70 |
| Lipids | max 15 | 12-14 |
| Protein | max 8 | 5-7 |
| Ash | max 10 | 5-9 |
| Dry matter | min 92 | 94-97 |

A further preferred source of glucan is MacroGard®AquaSol, which has the following composition:

| Component | % of dry matter | Typical range % |
|---|---|---|
| β-1,3/1,6-Glucan | min 95 | 96-99 |
| Lipids | max 1 | 0-1.0 |
| Ash | max 2 | 0.1-1.0 |
| Protein | max 1 | 0-1.0 |

Other MacroGard® products include MacroGard® Immersion Grade, MacroGard® Adjuvant, and MacroGard® F1 Suspension. MacroGard® Feed Ingredient is particularly preferred.

PatoGard® and MacroGard® (various types) are both suitable and generally preferred for all the methods and uses described herein.

A large number of plant protein sources may be used in connection with the present invention. The main reason for using plant proteins in the animal feed industry is to replace more expensive protein sources, like animal protein sources. Another important factor is the danger of transmitting diseases thorough feeding animal proteins to animals of the same species. Examples for plant protein sources include, but are not limited to, protein from the plant family *Fabaceae* as exemplified by soybean and peanut, from the plant family Brassiciaceae as exemplified by canola, cottonseed, the plant family *Asteraceae* including, but not limited to sunflower, and the plant family Arecaceae including copra. These protein sources, also commonly defined as oilseed proteins can be fed whole, but they are more commonly fed as a by-product after oils have been removed. Other plant protein sources include plant protein sources from the family Poaceae, also known as *Gramineae,* like cereals and grains especially corn, wheat and rice or other staple crops such as potato, cassava, and legumes (peas and beans), some milling by-products including germ meal or corn gluten meal, or distillery/brewery by-products. The most preferred proteins according to the present invention are soybean proteins and sunflower proteins from the plant families *Fabaceae* and *Asteraceae.*

In the fish farming industry the major fishmeal replacers with plant origin reportedly used, include, but are not limited to, soybean meal (SBM), maize gluten meal, Rapeseed/canola (*Brassica* sp.) meal, lupin (*Lupinus* sp. like the proteins in kernel meals of dehulled white (*Lupinus albus*), sweet (*L. angustifolius*) and yellow (L. luteus) lupins, Sunflower (Helianthus *annuus*) seed meal, crystalline amino acids; as well as pea meal (*Pisum sativum*), Cottonseed (*Gossypium* sp.) meal, Peanut (groundnut; Arachis *hypogaea*) meal and oilcake, soybean protein concentrate, corn (*Zea mays*) gluten meal and wheat (*Triticum aestivum*) gluten, Potato (*Solanum tuberosum* L.) protein concentrate as well as other plant feedstuffs like Moringa (*Moringa oleifera* Lam.) leaves, all in various concentrations and combinations.

The protein sources may be in the form of non-treated plant materials and treated and/or extracted plant proteins. As an example, heat treated soy products have high protein digestibility. Still, the upper inclusion level for full fat or defatted soy meal inclusion in diets for carnivorous fish is between an inclusion level of 20 to 30%, even if heat labile antinutrients are eliminated. In fish, soybean protein has shown that feeding fish with protein concentration inclusion levels over 30% causes intestinal damage and in general reduces growth performance in different fish species. In fact, most farmers are reluctant to use more than 10% plant proteins in the total diet due to these effects.

The present invention solves this problem and allows for plant protein inclusion levels of up to 40 or even 50%, depending on, amongst other factors, the animal species being fed, the origin of the plant protein source, the ratio of different plant protein sources, the protein concentration and the amount, origin, molecular structure and concentration of the glucan and/or mannan. More preferably, the plant protein inclusion levels are up to 40%, preferably up to 20 or 30 %. Typically the plant protein present in the diet is between 5 and 40%, preferably between 10 or 15 and 30%. These percentages define the percentage amount of a total plant protein source in the animal feed or diet, this includes fat, ashes etc. The next table illustrates how such sources are not pure protein. Preferred pure protein levels are up to 30%, typically up to 20%, preferably 5-25%. The inclusion level of the glucan and/or mannan-comprising source PatoGard® as used in the present invention was 2000 mg/kg diet and for MacroGard® 1000 mg/kg diet. Much higher levels of up to several times this amount, e.g. 2-10 times, may be used in the present invention. Thus, depending on the animal to be treated, their age and health, the mode of administration etc., different concentrations of mannan/glucan may be used.

The proportion of plant protein to other protein (e.g. fish protein) in the total feed or diet is 5:95 to 95:5, preferably 15:85 to 50:50, more preferably 25:75 to 45:55.

The above figures are particularly appropriate for feeding to fish. Mammalian diets according to the invention may include less plant protein, e.g. up to 25%, preferably 5-20%.

Many different types of plant products are marketed and used in the animal feed industry. As an example, soy protein products may be cooked full-fat soybeans, Expeller Extracted soybean meal (SBM), Solvent Extracted SBM, Dehulled SBM, Soy Protein Concentrates or Soy Isolates, to mention a few.

One aim and benefit of the present invention, particularly related to the use of the present invention in the fish farming industry, is to replace part or all of the fish meal in the diet with vegetable protein sources. Typical compositions of fish meal and vegetable protein ingredients currently used in commercial feeds for fish (% of dry matter) are as follows:

| Protein source | Protein | Oil | Starch | NSP | Sugars |
|---|---|---|---|---|---|
| Fish meal | 78 | 12 | - | - | - |
| Full fat soy | 42 | 21 | 3 | 18 | 11 |
| Hulled and defatted soy | 57 | 1 | 3 | 23 | 14 |
| Soy protein concentrate | 68 | 1 | 7 | 19 | 2 |
| Extracted sunflower | 40 | 3 | 2 | 33 | 5 |
| Corn gluten | 67 | 2 | 21 | 3 | 1 |
| Wheat gluten | 85 | 6 | 7 | - | - |
| NSP = non-starch polysaccharides | | | | | |

This table describes the amount of protein in the various "protein sources". As an example, fish meal has a protein content of 78%. Thus the amount of a protein source present is not the same as the actual protein content.

Without being bound to any theory, it is believed that a possibility for the mode of action might be that the mannan and/or glucan components interact with antinutritional factors from the protein sources and neutralize these gut-toxic components. Lectins are carbohydrate binding proteins that are present in most plants, especially seeds and tubers like cereals, potatoes, and beans. Their primary biological function is to protect the plant from infections, primarily fungi, but they have a possible role also to deter animals from eating the plant. Lectins present in major plant protein feeds, such as soybeans, are resistant to cooking and they are not degraded by proteolytic enzymes present in the digestive tract of warm-blooded animals or fish.

According to the present invention is provided a composition comprising an effective amount of a combination of one or more plant proteins and a glucan and/or a mannan together with a pharmaceutically or veterinary acceptable diluent, excipent or carrier. Such compositions have the utilities described above and in particular have utility in the treatment, including prophylactic treatment, of ectoparasitic infection or infestation of an animal, particularly a fish.

Ectoparasites are in general defined as parasites that live on or in the skin but not within the body. Examples are, but not limited to, the class *Insecta* including *Exopterygota* (like lice), *Endopterygota* like *Siphonaptera* (like fleas), *Diptera* (like true flies), the class *Acarina* (like ticks), from the orders *Mesostigmata, Prostigmata* and *Sarcoptiformes.*

The compositions and feed regimens of the present invention can be used in a wide variety of applications to prevent and/or inhibit weight loss and ectoparasite infections in animals or treat animals experiencing' weight loss or being infected with ectoparasites. For example, the synergistic composition with focus on weight loss can be used as a feed additive in fish farming, as well as farming of mammals like pigs, cows or horses. The fish farming industry, including of wild fish species and/or aquarium species, is a suitable environment for use with regard to the focus on treatment of ectoparasites such as fish lice. Ectoparasitic control is also useful in mammalian farming.

The formulations and feed regimens described herein are also of utility in the treatment of diarrhoea. Such a treatment includes a relative reduction in diarrhoea as compared to that seen with comparable feeds which are not in accordance with the invention. Diarrhoea can be assessed based on the amount of dry matter in faeces. Treatment also includes prevention, the feeds preventing an otherwise expected level of diarrhoea.

The formulations and feed regimens described herein are also of utility in the treatment of bowel disease and in improving bowel health. Relevant bowel diseases and conditions will typically be inflammatory and include Inflammatory Bowel Disease (IBD) which has been identified as such in various animals, in particular pets such as dogs and cats. Also, as discussed in the Examples, intestinal problems in fish caused by high plant protein diets, particularly affecting the colon, can be treated according to the present invention.

Frequently used carriers or auxiliaries include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. The pH and exact concentration of the various components of the composition,are adjusted according to routine skills. The compositions for veterinary use are preferably prepared and administered in dose units. The term "dose units" and its grammatical equivalents as used herein refer to physically discrete units suitable as unitary dosages for fish, each unit containing a predetermined effective and potentiating amount of at least one of the two active ingredients calculated to produce the desired therapeutic effect in association with the required physiologically tolerable carrier, e.g., a diluent or a vehicle. Dosage levels of the active compounds comprised in the synergetic composition of the present invention may vary.

By "an effective amount" is meant an amount of a compound, in a combination of the invention, effective to ameliorate the symptoms of, or ameliorate, treat,' prevent, delay the onset of or inhibit the progression of an infection or disease. Ultimately, the attending veterinarian will decide the appropriate amount and dosage regimen. The "effective amount" of the active ingredients that may be combined with the carrier materials to produce a single dosage will vary depending upon the host treated and the particular mode of administration. It will be understood, however, that the specific dose level for any particular animal will depend upon a variety of factors including the activity of the specific formulation employed, the site of infection, the infecting pathogen, the age, body weight, general health, sex, diet, time of administration, route of administration, rate of excretion, the duration of the treatment, the nature of concurrent therapy (if any), the severity of the particular disease undergoing treatment, the manner of administration and the judgment of the prescribing veterinarian.

The compositions according to the invention may be presented in the form of an article or carrier such as a tablet, coated tablet, lozenges, troches, syrups or elixirs, liposomes, powder/talc or other solid, solution, emulsions, suspension, liquid, spray, gel, drops, aerosol, douche, ointment, foam, cream, gel, paste, microcapsules, controlled release formulation, sustained release formulation or any other article or carrier which may possible or useful in light of the, at any give point in time and intended, preferred mode of administration.

The composition of the present invention may be provided alone or in combination with other medicaments to provide an operative combination. It is intended to include any chemically compatible combination of pharmacologically-active agents, as long as the combination does not eliminate the activity of the composition of the invention.

It will be appreciated that the composition of the present invention may be administered as a ready-to-use combination product, or each part of the composition of the present invention, may be administered separately, sequentially or simultaneously. Therapy may be repeated intermittently while parasites are detectable or even when they are not detectable. It might be relevant to administer the components two weeks prior to the expected challenge and/or for several weeks after the challenge. Continuous use is also possible.

The compositions may include one, two or several different plant proteins and one or more glucans and/or one or more mannan moieties, preferably at least one glucan and at least one mannan. The glucan and mannan combined will typically make up no more than 8%, preferably no more than 5%, more preferably no more than 2% of the total formulation or diet.

Where reference is made herein to *Asteraceae*, *Fabaceae* or other plant proteins, unless otherwise clear from the context, it should be understood that *Asteraceae, Fabaceae* or other plant meal may be used. In a further aspect, other components of the *Asteraceae* meal than the protein part may be used in place of the *Asteraceae* ' protein in various formulations and methods described herein.

The veterinary compositions include those adapted for enteral including oral administration, external application, like tablets, powders, granules or pellets for admixture with feed stuffs.

The subject for use of the compositions according to this invention, can be a non-human private, or other mammal, such as but not limited to dog, cat, horse, cow, pig, turkey, goat, monkey, chicken, rat, mouse, and sheep; as well as avian species, and aquatic animals, preferably fish.

For the purpose of this speci;fication it will be clearly understood that the word "comprising" means "including but not limited to", and that the word "comprises" have a corresponding meaning. Therefore the words "comprise", "comprises", and "comprising" are to be interpreted inclusively rather than exclusively.

As used herein and in the appended claims, the singular forms .lla," "an," and "the" include plural reference unless the context clearly dictates otherwise.

Unless defined otherwise, all technical and scientific terms and any acronyms used herein have the same meanings as commonly understood by one of ordinary skill in the art in the field of the invention.

The invention will now be further described in the following Examples and with reference to the figures in which:

Figure 1 shows the daily dry matter intake in gram including the different diets used in the present invention in relation to temperature.

Figure 2 describes the total dry matter intake in gram including the different diets used in the present invention in relation to temperature.

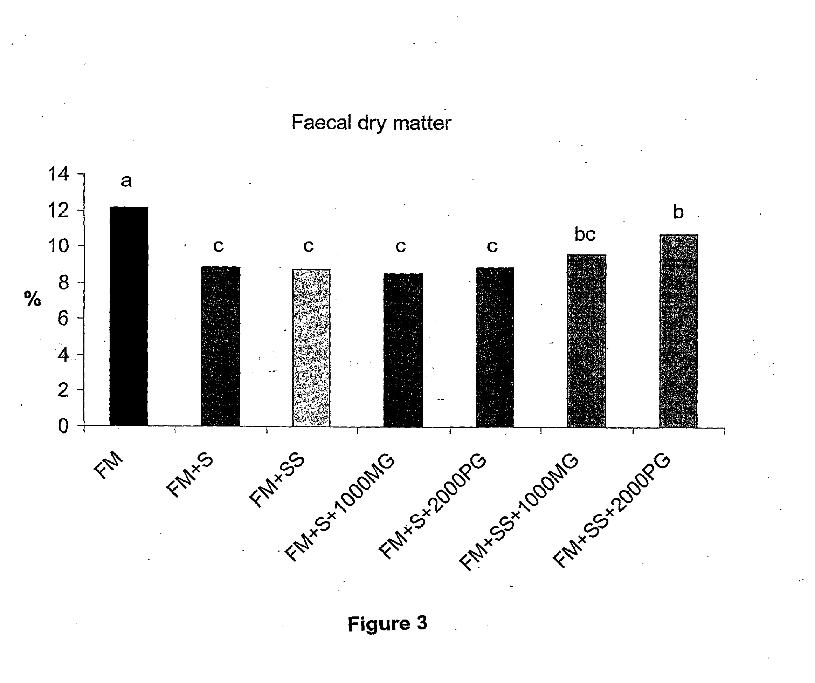

Figure 3 is a graph showing the percentage of dry matter in the faeces of Atlantic salmon, this being a good indicator of diarrhoea.

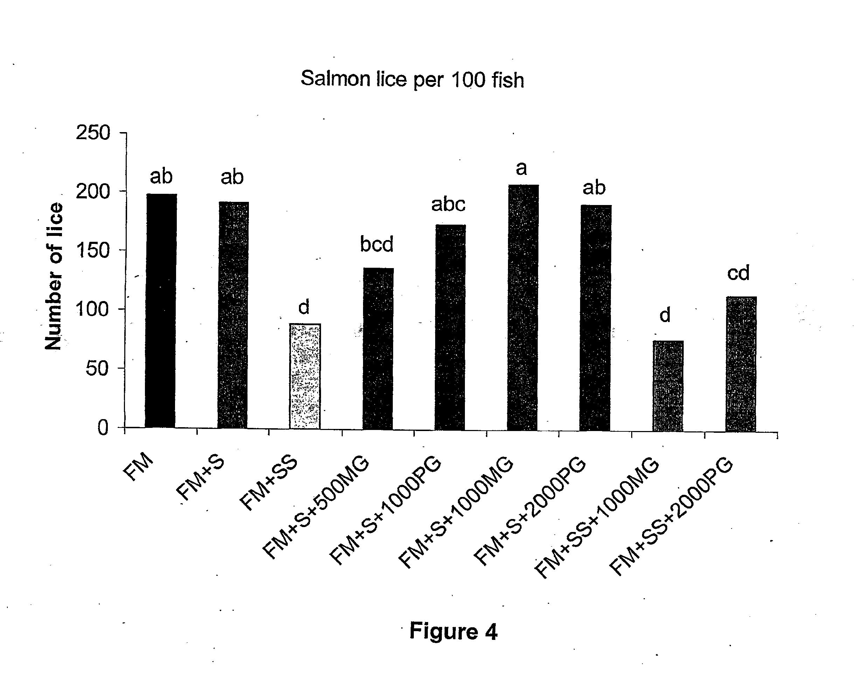

Figure 4 is a graph showing the number of lice per 100 Atlantic salmon fed a range of diets according to the invention and control diets.

The following examples are intended to be illustrative of the present invention and to teach one of ordinary skill in the art to make and use the invention. These examples are not intended to limit the invention in any way.

EXAMPLES

Example 1

1. Ingredients and diets

The formulation and composition of the diets is given in Tables 1 and 2, respectively. A standard fish meal based control diet (FM), a high-vegetable diet with 13.2% extracted and toasted soybean meal [SBM] and 13.5% extracted sunflower meal [SFM] (FM+SS), and a high-vegetable diet with 29.9% soybean meal (FM+S) were manufactured by high-pressure moist extrusion by Skretting (Averøy, Norway). The particle size was 6 mm, and all diets were dried prior to coating with fish oil.
Prior to coating with oil, batches of the basis FM+SS diet was first coated with 1000 mg of MacroGard® (FM+SS+1000MG) or 2000 mg PatoGard® (FM+SS+2000PG) per kg diet. Likewise, batches of the basis FM+S diets was pre-coated 500 (FM+S+500MG) or 1000 (FM+S+1000MG) mg MacroGard® or 1000 (FM+S+1000PG) or 2000 (FM+S+2000PG) PatoGard® per kg diet. This gave a series of nine experimental diets.

Table 1. Formulation of the experimental diets.

| Diet code | FM-control | FM + SBM + SFM (SS) | FM + SBM (S) |
|---|---|---|---|
| | | | |
| Formula, g kg$^{-1}$ | | | |
| LT-fish meal | 525.0 | 300.0 | 242.0 |
| SBM | | 135.0 | 320.0 |
| SFM | | 135.0 | |
| Wheat gluten | 0.0 | | 10.0 |
| Wheat | 188.0 | 116.5 | 100.5 |
| Fish oil | 286.0 | 291.0 | 305.0 |
| Lysine | | 1.0 | 1.0 |
| Methionine | | 1.5 | 1.5 |
| MCP* | 1.0 | 20.0 | 20.0 |
| *Mono Calcium Phosphate | | | |

Table 2. Composition of the experimental diets

| Basic diet | FM | FM +S | FM + SS | FM + S | | FM + SS | | | |
|---|---|---|---|---|---|---|---|---|---|
| Added | MG | PG | MG | PG | MG | PG | | | |

(continued)

| Basic diet | FM | FM +S | FM + SS | FM + S | | FM + SS | | | |
|---|---|---|---|---|---|---|---|---|---|
| Dose, mg kg$^{-1}$ | 500 | 1000 | 1000 | 2000 | 1000 | 2000 | | | |
| | | | | | | | | | |
| Dry matter, g | 937.3 | 934.0 | 941.0 | 933.1 | 930.3 | 932.7 | 930.8 | 940.8 | 942.7 |
| Crude protein*, g | 385.0 | 348.5 | 340.9 | 347.3 | 346.0 | 351.0 | 345.8 | 341.2 | 359.5 |
| Lipid, g | 336.7 | 327.6 | 347.6 | 335.0 | 319.6 | 331.9 | 326.1 | 348.6 | 352.5 |
| Starch, g | 100.6 | 53.9 | 57.1 | 58.0 | 53.7 | 59.6 | 54.2 | 56.7 | 71.3 |
| Ash, g | 80.0 | 62.8 | 68.6 | 63.2 | 59.7 | 64.9 | 62.6 | 69.1 | 73.4 |
| Energy, MJ | 24.3 | 24.2 | 24.7 | 24.4 | 24.2 | 24.1 | 24.0 | 24.6 | 24.9 |
| *CP; N x 6.25 | | | | | | | | | |

### 2.2 Fish, rearing conditions, and sampling

Atlantic salmon (Salmo salar) were fed the experimental diets for a total of 69 to 71 feeding days. Prior to the experiment, the fish were fed commercial diets (Skretting AS, Stavanger, Norway). The experiment was initiated in week 25 and terminated in week 36 of 2006.

The water temperature varied from 12.3 to 17.4 ˚C during the course of the experiment, averaging 15.3 ˚C.

At the start of the experiment, 27 groups of salmon (679 g, 150 fish per group) were randomly distributed to 5 x 5 x 5 m$^3$ sea pens. Each diet was then allocated to three groups of fish in a triplicate randomised experimental design. The fish were continuously fed by electrically driven feeders, and uneaten feed was collected from underneath the pens and pumped up into wire mesh strainers as described by Einen (1999). The feeding rate was planned to be 15% in excess, and was adjusted according to the recorded overfeeding every three days as described by Helland et al. (1996).

The fish were weighed in bulk at the start of the experiment and on feeding day 70. At the final weighing a sufficient number of fish were also anesthetised with tricaine methanesulfonate (MS 222, Argent Chemical Laboratories Inc., Redmont, Wa, USA) and stripped as described by Austreng (1978) to collect faeces for digestibility estimation. The faecal samples were pooled per pen and immediately frozen at -20˚C.

Before the final weighing 20 fish per pen were weighed individually and sampled for counting of salmon and sea lice to evaluate degree of lice infestation. Following this, blood was collected from the caudal vein from of the fish 10 fish into heparinised vacutainers. Blood samples were kept on ice until centrifugation at 3000 rpm for 10 minutes. Plasma samples were aliquoted into eppendorf tubes, frozen on dry ice and stored at -20 ˚C.

Out of the 10 remaining fish, five fish were euthanised by a sharp cranial blow for sampling of tissue from the mid intestine, defined as the intestine from the most proximal to the most distal pyloric caeca, and distal intestine, defined as the region characterised by the transverse luminal folds and increased intestinal diameter to the anus. From each fish, 5 mm tissue samples were cut (a transverse cut relative to the length of the tract) from the central area of each intestinal section. These samples were placed and stored in phosphate-buffered formalin (4%, pH 7.2) for histological examination.

Before distributing fish to the experimental sea pen, 15 fish were sampled from the holding pen. These fish were euthanised in water with a lethal concentration of MS 222, weighed individually,' and frozen immediately at -20˚C as three pooled samples of five fish. After feeding day 70 the fish were fasted for .two days before this procedure was repeated, sampling five fish per pen. These pooled samples were ground frozen and homogenised for analyses of chemical composition.

### 2.3 Calculations

Crude protein (CP) was calculated as N x 6.25. Protein was estimated after hydrolysing the protein for amino acid analysis as the sum of dehydrated amino acids (as when peptide-bound). Thermal-unit growth coefficient (TGC) was calculated according to Iwama and Tautz (1981), modified by Cho (1992), as: TGC = $(W_1^{1/3}-W_0^{1/3})$ x $(\Sigma D˚)^{-1}$, where $W_0$ and $W_1$ are the initial and final weights (pen means), respectively, and $\Sigma D˚$ is the thermal sum (feeding days x average temperature, ˚C). Feed intake was estimated by subtracting uneaten feed from fed feed on a dry matter basis. Recovery of uneaten feed was estimated as described by Helland et al. (1996), and the recorded uneaten feed was corrected for dry matter losses during feeding and collection. Apparent digestibility was estimated by the indirect method, as described by Maynard and Loosli (1969), using $Y_2O_3$ as an inert marker (Austreng et al., 2000).

2.4 Chemical analyses and histological examination

Homogenised fish were freeze-dried (Hetosicc Freeze drier CD 13-2 HETO, Birkerød, Denmark) and analysed for dry matter (105˚C to constant weight), ash (combusted at 550 ˚C to constant weight), nitrogen (Kjeltec Auto Analyser, Tecator, Höganäs, Sweden), and lipid (pre-extraction with diethylether and hydrolysis with 4 M HCl prior to diethylether extraction (Stoldt, 1952) in a Soxtec (Tecator) hydrolysing (HT-6) and extraction (1047) apparatus) .

Faeces were freeze-dried prior to analyses. Diets, and freeze dried faeces were analysed for dry matter, ash, nitrogen, lipid, starch (determined as glucose after hydrolysis by $\alpha$-amylase and amylo-glucosidase, followed by glucose determination by the "GODPOD method" (Megazyme, Bray, Ireland)), gross energy (Parr 1271 Bomb calorimeter, Parr, Moline, IL, USA) and yttrium (at Jordforsk, As, Norway, by inductivity coupled plasma (ICP) mass-spectroscopy, as previously described by Refstie et al. (1997)).

Formalin fixed intestinal tissue was routinely dehydrated in ethanol, equilibrated in xylene and embedded in paraffin according to standard histological techniques. Sections of approximately 5 $\mu$m were cut and stained with haematoxylin and eosin before examination under a light microscope. Intestinal morphology was evaluated according to the following criteria: (1) widening and shortening of the intestinal folds (2) loss of the supranuclear vacuolisation in the absorptive cells (enterocytes) in the intestinal epithelium; (3) widening of the central lamina propria within the intestinal folds, with increased amounts of connective tissue and (4) infiltration of a mixed leukocyte population in the lamina propria and submucosa. These are the characteristics of the condition previously described as soybean meal-induced enteritis in Atlantic salmon (Baeverfjord and Krogdahl, 1996).

2.5 Statistical analyses

The results were analysed by the General Linear Model procedure in the SAS computer software (SAS, 1985). Mean results per pen were subjected to one-way analysis of variance (ANOVA) with diet as the independent variable. Significant differences were indicated by Duncan's multiple range test. The level of significance was $P \leq 0.05$, and the results are presented as mean $\pm$ s.e.m. (standard error of the mean).

3. Results and discussion

3.1 Feed intake, growth, and feed efficiency

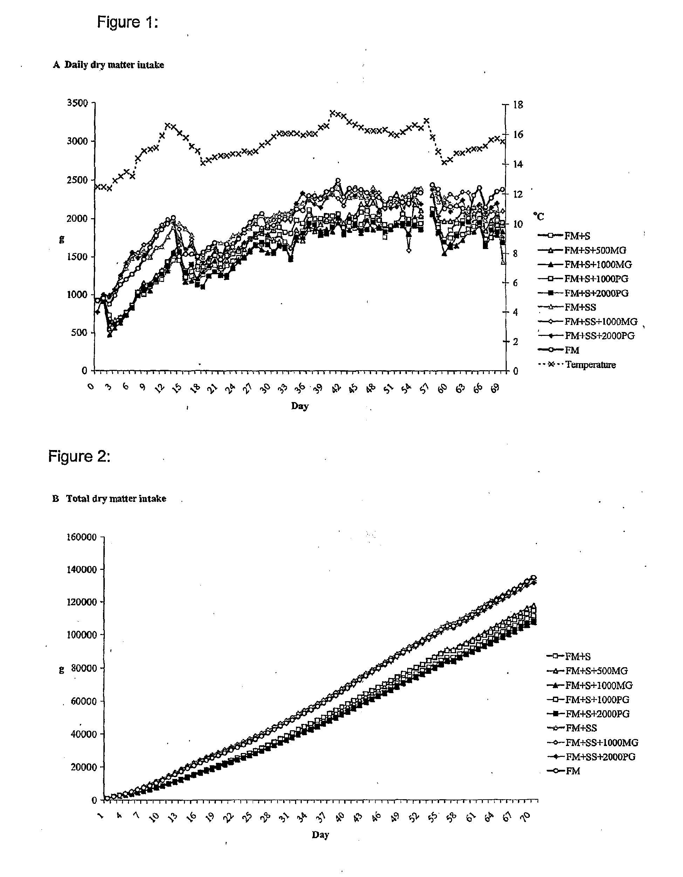

When comparing the feed intake in groups fed the fish meal (FM) control diet, the basis diet with 29.9 % extracted soybean meal (FM+S), and the basis diet with 13.2 % extracted soybean meal and 13.5 % extracted sunflower meal (FM+SS), the feed intake was when feeding FM and FM+SS than when feeding FM+S (Table 3 and Fig. 1). As shown by Fig 1, this was a consistent difference that lasted throughout the experiment. Furthermore, the feed intake dropped noticeably three days after introducing the fish to the FM+S diet, and the appetite in these groups was not normalised until the beginning of the second experimental week. As opposed to this, the feed intake in the groups fed the FM and FM+SS diets increased steadily in this period. Supplementation of MacroGard® (MG) or PatoGard® (PG) to the FM+S and FM+SS diets did not alter the feed intake.

The conversion of feed to growth was most efficient when feeding the FM diet, least efficient when feeding the FM+S diet, and intermediate when feeding the FM+SS diet (Table 3). Supplementing the FM+S diet with MG and PG actually resulted in poorer feed conversion when adding 500 mg MG (FM+S+500MG) or 2000 mg PG (FM+S+2000PG) per kg, while there was no effect when supplementing 1000 mg PG (FM+S+1000PG) or MG (FM+S+1000MG). As opposed to this, supplementing the FM+SS diet with 2000 mg PG per kg (FM+SS+2000PG) improved the feed conversion of this diet. Supplementing this diet with 1000 mg MG per kg (FM+SS+1000MG) did not affect the feed conversion efficiency.

Table 3. Intake (% of initial body weight, IBW), feed conversion ratio (FCR; g eaten per g BW increase) and feed efficiency ratio (FER; g BW increase per g feed eaten). when feeding the experimental diets for 70 days (n=3)

| | Feed (as is) | | Feed dry matter | | |
|---|---|---|---|---|---|
| Diet | Intake, % IBW | FCR | Intake, % IBW | FCR | FER |
| | | | | | |
| FM | 145.2[a] | 0.95[a] | 133.5[a] | 0.88[a] | 1.14[a] |
| FM+S | 119.5[bc] | 1.23[c] | 109.9[bc] | 1.13[c] | 0.89[c] |
| FM+SS | 144.7[a] | 1.04[b] | 132.6[a] | 0.95[b] | 1.05[b] |

(continued)

|  | Feed (as is) | | Feed dry matter | | |
|---|---|---|---|---|---|
| Diet | Intake, % IBW | FCR | Intake, % IBW | FCR | FER |
| FM+S+500MG | 127.1[b] | 1.33[e] | 116.9[b] | 1.23[e] | 0.82[e] |
| FM+S+1000PG | 124.2[bc] | 1.25[cd] | 113.7[bc] | 1.15[cd] | 0.87[cd] |
| FM+S+1000MG | 116.2[c] | 1.22[c] | 106.7[c] | 1.12[c] | 0.89[c] |
| FM+S+2000PG | 116.3[c] | 1.28[d] | 107.3[c] | 1.18[d] | 0.85[d] |
| FM+SS+1000MG | 146.0[a] | 1.03[b] | 133.1[a] | 0.94[b] | 1.07[b] |
| FM+SS+2000PG | 141.7[a] | 0.94[a] | 130.3[a] | 0.87[a] | 1.16[a] |
| <u>ANOVA:</u> | | | | | |
| P | <0.0001 | <0.0001 | <0.0001 | <0.0001 | <0.0001 |
| $\sqrt{MSE}$ | 4.5 | 0.03 | 4.2 | 0.02 | 0.02 |

Different superscripts [abcde] within column indicates significant differences as indicated by Duncan's Multiple Range Test (P<0.05).

Growth of the control groups (fed the FM diet) was within the expected range when considering the high water temperature. When comparing the groups fed FM, FM+S, and FM+SS, both weight gain and growth rate mirrored the feed conversion efficiency (Table 4). Supplementing the FM+S diet with MG or PG did not alter the growth.

The same was seen when supplementing the FM+SS diet with 1000 mg MG per kg. When supplementing this diet with 2000 mg PG, however, the fish grew faster and at a similar rate as when feeding the FM diet. Similar feed intake but improved feed conversion efficiency in fish fed the FM+SS+2000PG diet than in fish fed the FM+SS diet showed that this growth difference was caused by improved utilisation of the consumed nutrients for growth, and not by changed appetite.

The mortality in the experiment was low, and not affected by diet.

Table 4. Weight gain, growth rate estimated as Specific Growth rate (SGR) and Thermal-unit Growth Coefficient (TGC x 1000), and mortality after feeding the experimental diets for 70 days (n=3)

| Diet | Initial BW, g | Final BW, g | SGR %/d | TGC x 1000 | Mortality % |
|---|---|---|---|---|---|
| FM | 679 | 1716[a] | 1.32[a] | 2.95[a] | 0.22 |
| FM+S | 680 | 1346[c] | 0.97[cd] | 2.09[cd] | 0.44 |
| FM+SS | 679 | 1632[b] | 1.26[b] | 2.79[b] | 1.11 |
| FM+S+500MG | 679 | 1327[c] | 0.95[cd] | 2.04[cd] | 0.00 |
| FM+S+1000PG | 679 | 1354[c] | 0.99[c] | 2.12[c] | 0.22 |
| FM+S+1000MG | 679 | 1327[c] | 0.95[cd] | 2.04[cd] | 0.22 |
| FM+S+2000PG | 679 | 1299[c] | 0.93[d] | 1.98[d] | 0.22 |
| FM+SS+1000MG | 678 | 1649[b] | 1:26[b] | 2.81[b] | 0.67 |
| FM+SS+2000PG | 679 | 1707[a] | 1.32[a] | 2.95[a] | 0.44 |
| ANOVA: | | | | | |
| P | 0.9867 | <0.0001 | <0.0001 | <0.0001 | 0.6836 |
| $\sqrt{MSE}$ | 3 | 31 | 0.03 | 0.07 | 0.68 |

Different superscripts [abcd] within column indicates significant differences as indicated by Duncan's Multiple Range Test (P<0.05).

The FM+SS+2000PG-diet provides a better FCR compared to the FM-diet which is the standard diet. Compared to the FM+SS diet with an FCR of 1.04, the FM+SS+2000PG-diet improved the FCR to 0.94 which is a significantly better result which is due to the addition of the PatoGard®-product. Higher concentrations of MacroGard®, this was added at 500 and 1000 mg/kg diet as compared to PatoGard® at 2000 mg/kg diet, are anticipated to yield similar results.

3.2. Applicable plant protein concentrations

Table 5 shows a calculation of the amount of plant protein in the total protein content of the animal feed diet 2 as used in the present invention.

| Protein Source | Protein content in commercially available animal feeds (in % of total) | Amount of protein source used in diet 2 (in % of the total feed) | Total protein content in the diet (in % of the total feed) | Protein content in % of the total protein content in the feed |
|---|---|---|---|---|
| Fish meal source | 78 | 29.9 | 23,3 | 63 % |
| Soy protein source | 50 | 13.2 | 6,6 | 18% |
| Sunflower protein source | 40 | 13.5 | 5,4 | 15% |
| | | | | |
| Wheat protein source | 10 | 15 | 1,5 | 4% |
| Summarized protein content in the feed | | | 36,8 | 100 % |
| Total share of plant protein content in the feed | | | | 37 % |

Table 5 shows that 37% of the protein content of the total feed was from plant protein sources, while 63% was the usual fish meal protein source. This is a significant increase of possible additions of plant proteins to animal feed compared to the 10% limit as used in the farming industry and which is defined as a commercially acceptable figure. It is, without doubt possible to increase this concentration to a higher level by increasing the amount of the glucan- and/or mannan-comprising source.

3.3 Lice infestation

Table 6 shows different diets including the products MacroGard® and PatoGard® in relation to the number of lice infesting fish.

Table 6. % of the fish infested by salmon lice (*Lepeophteirus salmonis*) and sea lice (*Caligus elongatus*), number of lice per infested fish, and estimated number of lice per 100 fish after feeding the experimental diets for 70 days (n=3).

| Diet | Salmon lice | | | Sea lice | | |
|---|---|---|---|---|---|---|
| | Infested Fish % | Lice per infested fish | Lice per 100 fish | Infested Fish % | Lice per infested Fish | Lice per 100 fish |
| FM | 77.3[ab] | 2.54[a] | 197[ab] | 36.4 | 1.06 | 39 |
| FM+S | 77.3[ab] | 2.42[ab] | 191[ab] | 22.7 | 1.12 | 26 |
| FM+SS | 60.6[c] | 1.48[d] | 89[d] | 25.8 | 1.25 | 32 |
| FM+S+500MG | 72.7[abc] | 1.86[bcd] | 136[bcd] | 13.6 | 1.13 | 17 |
| FM+S+1000PG | 77.3[ab] | 2.24[abc] | 173[abc] | 19.7 | 1.07 | 21 |
| FM+S+1000MG | 84.8[a] | 2.43[ab] | 208[a] | 24.2 | 1.00 | 24 |

(continued)

| | Salmon lice | | | Sea lice | | |
|---|---|---|---|---|---|---|
| Diet | Infested Fish % | Lice per infested fish | Lice per 100 fish | Infested Fish % | Lice per infested Fish | Lice per 100 fish |
| FM+S+2000PG | 84.8[a] | 2.25[abc] | 191[ab] | 21.2 | 1.11 | 26 |
| FM+SS+1000MG | 43.9[d] | 1.75[cd] | 77[d] | 19.7 | 1.30 | 24 |
| FM+SS+2000PG | 68.2[bc] | 1.70[cd] | 115[cd] | 16.7 | 1.18 | 20 |
| ANOVA | | | | | | |
| P | <0.0001 | 0.0056 | 0.0009 | 0.2888 | 0.5800 | 0.6094 |
| $\sqrt{MSE}$ | 7.9 | 0.32 | 35 | 9.7 | 0.18 | 13 |

Different superscripts [abcd] within column indicates significant differences as indicated by Duncan's Multiple Range Test (P<0.05).

ANOVA = Analysis of Variance

The infestation with salmon lice was significantly reduced in groups fed diets containing 15% sunflower meal. This indicated that dietary extracted sunflower meal actually reduced salmon lice infestation.

When comparing the FM+SS diets, the frequency of salmon lice infested fish was reduced when supplementing this diet with 1000 mg MacroGard© per kg.

3.3 Diarrhoea, nutrient digestibility and retention

Feeding the FM+S and FM+SS diets generally resulted in lower dry matter content (i.e. more water) in the faeces, than when feeding the FM diet, vindicating diarrhoea (Table 7 below).

Table 7. Apparent digestibility of nutrients and retention of nitrogen and energy by the fish after feeding the experimental diets for 70 days (n=3).

| | Faecal dry | Apparent digestibility (%) of | | | | Retention | |
|---|---|---|---|---|---|---|---|
| (%) of | | | | | | | |
| Diet | matter, % Energy | Nitrogen | Lipid | Starch | Energy | Nitrogen | |
| FM | 12.2a | 71.0 | 84.5ab | 27.5b | 68.7 | 46.5c | 58.9 |
| FM+S | 8.8c | 75.1 | 77.3bc | 49.8a | 64.9 | 40.4d | 45.5 |
| FM+SS | 8.7c | 75.6 | 87.7a | 46.1a | 70.8 | 50.1b | 55.1 |
| FM+S+1000MG | 8.5c | 73.3 | 70.0c | 51.0a | 59.3 | 39.8d | 46.1 |
| FM+S+2000PG | 8.8c | 72.8 | 71.0c | 47.3a | 59.9 | 38.9d | 44.8 |
| FM+SS+1000MG | 9.5bc | 77.5 | 88.2a | 48.6a | 71.5 | 47.8bc | 55.1 |
| FM+SS+2000PG ANOVA: | 10.7b | 77.1 | 89.7a | 42.6a | | 55.5a | |
| P | <0.0001 | 0.27 | 0.0008 | 0.02 | | <0.0001 | |
| $\sqrt{MSE}$ | 0.7 | 3.4 | 5.2 | 7.2 | | 1.7 | |

Different superscripts [abcd] within column indicates significant differences as indicated by Duncan's Multiple Range Test (P<0.05).

When comparing the FM+SS diets, this was significantly ameliorated when adding 2000 mg kg-1 of PG, and the diarrhoeic condition also tended to improve when adding 1000 mg kg-1 of MG. No such effect og MG and PG was seen when added to the FM+S basis diet.

The apparent digestibility of nitrogen (i.e. crude protein) was not affected by diet (Table 7). Likewise, the apparent digestibility of starch was similar when feeding the FM+S and FM+SS diets, but was reduced when feeding the FM control diet in response to the much higher starch level in this diet. The lipid digestibility was, however, reduced when feeding the FM+S diets, and this was not seen when feeding the FM+SS diets. The energy digestibility mirrored that of the lipid digestibility.

Diarrhoea and low lipid digestibility when feeding the FM+S diets was in line with previous results. The high lipid digestibility when feeding the FM+SS diets was unexpected, as these diets actually contained 15% SBM. This indicates that dietary SFM ameliorates negative digestive effects of dietary SBM in Atlantic salmon, and may even have a positive effect on the lipid digestion and/or absorption.

The apparent digestibility estimates were unexpectedly low. This may be because the inert digestibility marker (yttrium oxide) was coated on the feed particles post-extrusion. Thus, some of the marker may have been washed of the pellets while they were sinking through the water column in the pens before being eaten.

The retention of nitrogen (i.e. crude protein) and energy was lowest when feeding the FM+S diets (Table 7), in line with the low feed intake and subsequent low FER (high FCR) when feeding these diets. At low feed intake a relatively large proportion of the nutrients and energy is used for metabolic maintenance functions, and consequently a relatively small proportion is utilised for growth. The retention of nitrogen (crude protein) was also relatively low when feeding the FM control diet. This was because the crude protein content in this diet was high, when compared to the other diets, resulting in more catabolism for energy and/or conversion of dietary amino acids to sugar and lipid and, thus, less efficient utilisation of the protein for muscle growth.

When comparing the FM+SS diets, the nitrogen retention was higher than when feeding the FM and FM+S diets. Furthermore, it was significantly improved by adding 2000 mg kg-1 of PG to the FM+SS basis diet. As the feed intake was similar with all FM+SS diets, this supports the higher FER (lower FCR) when feeding the FM+SS+2000PG diet, demonstrating more efficient utilisation of the dietary protein for growth as a result of the PG supplement.

Example 2

Experimental design:

The effect of the feed β 1,3/1,6-glucan product MacroGard® and the hydrolysed yeast cell / whole yeast cell product PatoGard™ were tested with animal feed products with an unconventionally high concentration of plant proteins. The purpose of the high-protein feed was to generate conditions in the intestines and to evaluate the effect of the products MacroGard® and PatoGard™ in the prevention and treatment of such conditions. The fish used in these trials was atlantic salmon (*Salmo salar*).

A high content of plant proteins over 15% leads generally to reduced colon health in fish as fish are not used to this kind of high protein diet. In this trial the total protein content was increased to 32% thus leading to detrimental effects on the fish colon. For purpose of investigating the effects of different plant proteins, both soy and sunflower proteins were used and compared to the golden standard being an easy to digest fish meal product.

The trial included in total nine' different groups. The different groups were fed with PatoGard™ and MacroGard® (65 %, β-1,3/1,6-glucan). The control groups received only the prepared high-plant protein containing animal feed. The distribution can be seen in Table 8 below.

Feed 1 FM: Feed with fishmeal only
Feed 2: FMS: Feed with fish meal and 32% soy
Feed 3: FMSPG: Group 2 feed with 2000 mg PatoGard™
Feed 4: FMSMG: Group 2 feed with 1000 mg MacroGard®
Feed 5: FMSS: Feed with fish meal, 15% soy and 15% sunflower meal
Feed 6: FMSSPG: Group 5 feed with 2000 mg PatoGard™
Feed 7: FMSSMG: Group 5 feed with 1000 mg MacroGard®

The seven groups consisted of 150 fish which were bred in 5x5x5 meter trail basins in the sea. The groups were fed with the respective feeds for 71 days. After that period the fish were measured and weighed and tissue samples were taken from the intestines of 27 randomly chosen fish. Tissue alterations were registered by using a standard method (Bæverfjord og Kroghdahl) and classified after the Uran-score. The score focuses on (1) the presence and size of supranuclear vacuoles (2) degree of widening of the lamina propria of simple folds (3) amount of connective tissue between the base folds and stratus compactum of the and (4) degree of thickening of the mucosal folds. Every check point is classified on a scale from 1-5 where 1 means undamaged and 5 is a lethal damage.

**Results and discussion:**

Table 8: Intestinal health with group 1-7 feed measured by using the Uran score

| Feed group | Defintion feed | Uran-score |
|---|---|---|
| 2 | FMS | 3,68 |
| 3 | FMSMG | 3,43 |
| 4 | FMSPG | 3,37 |
| 5 | FMSS | 2,05 |
| 6 | FMSSMG | 1,61 |
| 7 | FMSSPG | 1,23 |
| 1 | FM | 1,13 |

Detrimental intestinal health in group 2 and 5 fish feed was considerably reduced by adding Patogard™ and MacroGard® to the feed (groups 3, 4, 6 and 7). The addition of Patogard™ gave a significant reduction in relation to the feeds including soy alone or a combination of soy and sunflower. These results show clearly that both Patogard™ and MacroGard® eliminate the detrimental effect of plant proteins in the feed and lead thus to an improved bowel health.

References

Austreng, E., 1978. Digestibility determination in fish using chromic oxide marking and analysis of contents from different segments of the gastrointestinal tract. Aquaculture 13, 265-272.

Austreng, E., Storebakken, T., Thomassen, M.S., Refstie, S., Thomassen, Y., 2000. Evaluation of selected trivalent metal oxides as inert markers used to estimate apparent digestibility in salmonids. Aquaculture 188, 65-78.

Cho, C.Y., 1992. Feeding systems for rainbow trout and other salmonids with reference to current estimates of energy and protein requirements. Aquaculture 100, 107-123.

Einen, O., Mørkøre, T., Rørå, A.M.B., Thomassen, M.S., 1999. Feed ration prior to slaughter - a potential tool for managing product quality of Atlantic salmon (Salmo salar). Aquaculture 178, 149-169.

Helland, S.J., Grisdale-Helland, B., Nerland, S., 1996. A simple method for the measurement of daily feed intake of groups of fish in tanks. Aquaculture 139, 157-163.

Iwama, G.K., Tautz, A.F., 1981. A simple growth model for salmonids in hatcheries. Can. J. Fish. Aquat. Sci. 38, 649-656.

Maynard, L.A., Loosli, J.K., 1969. Animal Nutrition, 6th edn. McGraw-Hill, New York, NY, USA.

Refstie, S., Helland, S., Storebakken, T., 1997. Adaptation to soybean meal in diets for rainbow trout, Oncorhynchus mykiss. Aquaculture 153, 263-272.

SAS, 1985. SAS/STAT® Guide for personal computers, Version 6 Ed. Cary, N.C., SAS Institute Inc., 378 pp.

Stoldt, W., 1952. Vorschlag zur Vereinheitlichung der Fettbestimmung in Lebensmitteln. Fette, Seifen, Anstrichm. 54, 206-207.

Embodiments of the invention are also described in the claims of the International application as filed:

1. An animal feed composition comprising protein from the family *Fabaceae* and protein from the family Asteraceae.

2. The composition as claimed in claim 1 further comprising glucan.

3. The composition as claimed in claim 1 or 2 further comprising fish meal.

4. The composition as claimed in any one of claims 1 to 3 which is a fish feed composition.

5. The composition according to any preceding claim wherein said Fabaceae protein is soy protein.

6. The composition according to any preceding claim wherein said Asteraceae protein is *Helianthus* protein.

7. The composition according to claim 2 wherein said glucan is derived from yeast.

8. The composition according to claim 7 wherein the yeast is *Saccharomyces cerevisiae.*

9. The composition according to claim 7 or 8 wherein said glucan is comprised mainly of (1,3) linked glucopyranose sub-units, having less than 10% (1,6) linked glucopyranose sub-units.

10. Use of a composition as claimed in any preceding claim for increasing or stabilising weight in an animal.

11. Use of a composition as claimed in claim 1 together with a separately formulated glucan as defined in any one of claims 2 or 7 to 9 for increasing or stabilising weight in an animal.

12. A composition as claimed in any one of claims 1 to 9 for use in veterinary therapy.

13. A composition as claimed in claim 12 for use in treating a condition selected from the group comprising, an ectoparasitic infection or infestation, diarrhoea and bowel disease.

14. A composition as claimed in claim 1 for use in treating a condition selected from the group comprising, an ectoparasitic infection, diarrhoea and bowel disease, wherein a separately formulated glucan as defined in any one of claims 2 or 7 to 9 is co-administered to said animal.

15. A composition as claimed or defined in any preceding claim which comprises 10-30% of protein from the family Fabaceae and 10-30% of protein from the family *Asteraceae.*

16. A composition as claimed in any one of claims 1-9 further comprising mannan.

17. Use of a composition as claim in claim 1 together with a separately formulated mannan for increasing or stabilising weight in an animal.

18. A composition as claimed in claim 1 for use in treating a condition.in an animal'selected from the group comprising, an ectoparasitic infection or infesatation, diarrhoea and bowel disease, wherein a separately formulated mannan is co-administered to said animal.

19. A use or composition as claimed in claim 17 or 18 wherein glucan is formulated with the mannan.

20. Use of protein from the family Asteraceae to ameliorate the side effects of plant Fabaceae protein in an animal feed or diet, wherein the protein from the family *Asteraceae* is administered to an animal together with or separately from said Fabaceae protein.

21. A method of ameliorating the side effects of Fabaceae protein in an animal feed or diet, which method comprises administering an effective amount of Asteraceae protein to an animal together with or separately from said Fabaceae protein.

22. A product comprising (a) glucan and/or mannan and (b) a mixture of plant proteins from Fabaceae and *Asteraceae,* as a combined preparation for simultaneous, separate or sequential administration to an animal for stabilising or enhancing weight of said animal, treating an ectoparasitic infection or infestation in said animal, treating diarrhoea or treating bowel disease.

23. Use of *Asteraceae* meal or any component thereof to ameliorate the side effects of *Fabaceae* protein in an animal feed or diet, wherein the Asteraceae meal or component is administered to said animal together with or separately from said Fabaceae protein.

24. A veterinary composition comprising protein from the family *Fabaceae* and protein from the family *Asteraceae* for use in treating a condition in an animal selected from the group comprising an ectoparasitic infection or infestation, diarrhoea or bowel disease.

25. A composition as claimed in claim 24 wherein said composition further comprsies or is co-adminsitered with glucan or mannan.

26. Asteraceae meal or any component thereof, optionally co-administered with glucan or mannan, for use in treating diarrhoea or bowel disease.

27. Use of Asteraceae meal or any.component thereof, optionally co-administered with glucan or mannan, for increasing or stabilising weight in an animal.

**Claims**

1. Glucan or mannan for use in ameliorating the side-effects of *Fabaceae* protein in an animal diet, whereby the glucan or mannan is administered to an animal together with or separately from said *Fabaceae* protein.

2. The glucan or mannan for use according to claim 1, wherein said *Fabaceae* protein is present in the form of meal.

3. The glucan or mannan for use according to any claim 1 or claim 2 , wherein said *Fabaceae* protein is soy protein.

4. The glucan or mannan for use according to any one of claims 1 to 3, wherein said glucan is derived from yeast.

5. The glucan or mannan for use according to claim 4, wherein said yeast is *Saccharomyces cerevisiae.*

6. The glucan or mannan for use according to any one of claims 1 to 5, wherein said glucan has a backbone made of β-(1,3)-linked glucopyranose sub-units.

7. The glucan or mannan for use according to claim 6, wherein said glucan comprises branches of β-(1,3)-linked glucopyranose sub-units attached to the backbone by a β-(1,6)-linkage.

8. The glucan or mannan for use according to any one of claims 1 to 7, wherein said glucan has less than 10% β-(1,6)-linked glucopyranose sub-units.

9. The glucan or mannan for use according to any one of claims 1 to 8, wherein both a glucan and a mannan are administered.

10. The glucan or mannan for use according to any one of claims 1 to 9, wherein said animal is a fish.

11. The glucan or mannan for use according to any one of claims 1 to 10, wherein the side effect of *Fabaceae* protein in the animal diet is diarrhoea or bowel disease.

Figure 1:

A  Daily dry matter intake

Figure 2:

B  Total dry matter intake

## Faecal dry matter

**Figure 3**

Salmon lice per 100 fish

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **AUSTRENG, E.** Digestibility determination in fish using chromic oxide marking and analysis of contents from different segments of the gastrointestinal tract. *Aquaculture,* 1978, vol. 13, 265-272
- **AUSTRENG, E. ; STOREBAKKEN, T. ; THOMASSEN, M.S. ; REFSTIE, S. ; THOMASSEN, Y.** Evaluation of selected trivalent metal oxides as inert markers used to estimate apparent digestibility in salmonids. *Aquaculture,* 2000, vol. 188, 65-78
- **CHO, C.Y.** Feeding systems for rainbow trout and other salmonids with reference to current estimates of energy and protein requirements. *Aquaculture,* 1992, vol. 100, 107-123
- **EINEN, O. ; MØRKØRE, T. ; RØRÅ, A.M.B. ; THOMASSEN, M.S.** Feed ration prior to slaughter - a potential tool for managing product quality of Atlantic salmon (Salmo salar). *Aquaculture,* 1999, vol. 178, 149-169
- **HELLAND, S.J. ; GRISDALE-HELLAND, B. ; NERLAND, S.** A simple method for the measurement of daily feed intake of groups of fish in tanks. *Aquaculture,* 1996, vol. 139, 157-163
- **IWAMA, G.K. ; TAUTZ, A.F.** A simple growth model for salmonids in hatcheries. *Can. J. Fish. Aquat. Sci.,* 1981, vol. 38, 649-656
- **MAYNARD, L.A. ; LOOSLI, J.K.** Animal Nutrition. McGraw-Hill, 1969
- **REFSTIE, S. ; HELLAND, S. ; STOREBAKKEN, T.** Adaptation to soybean meal in diets for rainbow trout, Oncorhynchus mykiss. *Aquaculture,* 1997, vol. 153, 263-272
- SAS/STAT® Guide for personal computers. SAS Institute Inc, 1985, 378
- **STOLDT, W.** Vorschlag zur Vereinheitlichung der Fettbestimmung in Lebensmitteln. *Fette, Seifen, Anstrichm.,* 1952, vol. 54, 206-207